# EUROPEAN PATENT SPECIFICATION

(11) **EP 2 113 559 B1**
(45) Date of publication and mention of the grant of the patent: **09.03.2016**
(21) Application number: 08718466.9
(22) Date of filing: 08.02.2008
(51) Int. Cl.: A01N 43/16, A01N 63/02

(54) **USE OF CHITOSAN FOR INCREASING SPORULATION OF FUNGI**
VERWENDUNG VON CHITOSAN FÜR ERHÖHTE SPORULATION VON PILZEN
UTILISATION DU CHITOSANE POUR AUGMENTER LA SPORULATION DE CHAMPIGNONS

(30) Priority: 22.02.2007 ES 200700461
(43) Date of publication of application: 04.11.2009
(73) Proprietor: Universidad De Alicante, 03690 Alicante (ES)
(72) Inventor: PALMA GUERRERO, Javier, E-03690 Alicante (ES); LÓPEZ LLORCA, Luis Vicente, E-03690 Alicante (ES); JANSSON, Hans-Börje, E-03690 Alicante (ES); SALINAS CALVETE, Jesús, E-03690 Alicante (ES); GÜERRI AGULLÓ, Berenice, E-03690 Alicante (ES)
(74) Representative: Temino Ceniceros, Ignacio
(86) International application number: PCT/ES2008/070019
(87) International publication number: WO 2008/102044

(56) References cited:
- EP-A1- 1 332 676
- JP-A- 2000 224 983
- KR-A- 20030 079 482
- DATABASE WPI Week 199409 Thomson Scientific, London, GB; AN 1994-068476 XP002685893, & JP 6 014765 A (SNOW BRAND MILK PROD CO LTD) 25 January 1994 (1994-01-25)
- SILVIA BAUTISTA-BANOS AND OTHERS: "Effect of Chitosan on in vitro Development and Morphology of two isolates of colletotrichum gloeosporioides (Penz.) Penz. and Sacc.", REVISTA MEXICANA DE FITOPATOLOGIA, vol. 23, no. 1, 15 March 2005 (2005-03-15) , pages 62-67, XP002685894, Obregon, Mexico ISSN: 0185-3309
- SILVIA BAUTISTA-BANOS AND OTHERS: "Growth inhibition of selected fungi by chitosan and plant extracts.", REVISTA MEXICANA DE FITOPATOLOGÍA, vol. 22, no. 2, 27 February 2004 (2004-02-27), pages 178-186, XP002685895, Obregon, Mexico
- DATABASE BIOSIS [Online] BIOSCIENCES INFORMATION SERVICE, PHILADELPHIA, PA, US; March 1998 (1998-03), REDDY M V BHASKARA ET AL: "Effect of chitosan on growth and toxin production by Alternaria alternata f. sp. lycopersici", XP002685896, Database accession no. PREV199800262602 & BIOCONTROL SCIENCE AND TECHNOLOGY, vol. 8, no. 1, March 1998 (1998-03), pages 33-43, ISSN: 0958-3157
- HEGEDUS D D ET AL: "BEAUVERIA-BASSIANA SUBMERGED CONIDIA PRODUCTION IN A DEFINED MEDIUM CONTAINING CHITIN TWO HEXOSAMINES OR GLUCOSE", APPLIED MICROBIOLOGY AND BIOTECHNOLOGY, SPRINGER VERLAG, BERLIN, DE, vol. 33, no. 6, 1 January 1990 (1990-01-01), pages 641-647, XP009164089, ISSN: 0175-7598
- PACHECO LOPEZ N. ET AL.: 'Efecto del quitosano y enzimas de Lecanicillium lecanii sobre la germinacion de esporas de Aspergillus niger utilizando procesamiento of imagenes' XXVIII CONGRESO NACIONAL DE HISTOLOGIA, MEXICO D.F., [Online] 25 October 2004 - 29 October 2004, XP008133761 Retrieved from the Internet: <URL:http://www.izt.uam.mx/mglo/Memorias%20 XXVIII%20CNH.pdf>
- MAGUNACELAYA J. ET AL.: 'Quitosano (Biorend) como complemento a la accion de productos nematicidas en vid de mesa y vinifera' XIV CONGRESO NACIONAL DE FITOPATOLOGIA, TALCA CHILE, [Online] 30 November 2004 - 03 December 2004, XP008133762 Retrieved from the Internet: <URL:http://www.alerce.inia.cl/sochifit/XIV .html>

## Description

### Field of Invention

This invention relates to a composition based on the use of chitosan to increase the sporulation of fungi, biological control agents.

### Background of the Invention

The use of natural organisms such as bacteria, viruses and fungi to control pests and diseases is of great interest. In particular, fungi are organisms with great potential as biological control agents because they have an extremely high reproductive capacity, a short generation time, and sometimes are very specific in its action, attacking only the host with which they have co-evolved. In addition, fungi have saprophytes phases in which they can survive without the host, and remain in the environment until the host appears again (12).

Nematophagous fungi are a group of fungal antagonists of nematodes (parasites of plants and animals) (18), therefore, they are good candidates for its use as biological control agents of parasite nematodes (31). They also have the ability to infect and colonize other organisms, including other fungi and plant roots (10).

*Pochonia chlamydosporia* is a good example of this type of fungus, which is used as a biological control agent against phytopathogenic nematodes (5, 11, 18), especially radicular nematodes. Furthermore, it is a good colonizer of the root of cereals (15) and various vegetables (3, 4), and can inhibit the growth of these pathogenic fungi in experiments *in vitro* (6, 9, 25).

Other nematophagous fungi of special interest are *Paecilomyces lilacinus, Lecanicillium lecanii* and *Pochonia rubescens,* which also inhibit various pathogens *in vitro* (6, 9, 23, 7).

Entomopathogenic fungi are a group of fungal pathogens of insects with numerous examples of its effectiveness in the suppression of insect pests, thus showing great potential as biological control agents (8). The genera *Beauveria, Metarhizium, Lecanicillium, Cordyceps* and *Paecilomyces* are the most important for their development as biological control agents against pests of plants (1).

For example, the entomopathogenic fungus *Beauveria bassiana,* in addition to its role in controlling plant pests, is capable of endophytic colonization of tissues (7, 29, 19) and to inhibit the growth of the phytopathogenic fungus *Penicillium vermoesenii* in experiments *in vitro* (1).

On the other hand, chitosan is a polymer of β-1-4 glucosamine, a partially deacetylated form of chitin. Chitosan can be obtained by chemical processes by subjecting chitin to the action of a highly concentrated alkaline medium, and temperatures above 60°C. Under these conditions the reaction of deacetylation takes place, consisting in the loss of the acetyl rest in the carbon 2 of the amide group of N-acetylglucosamine, obtaining an amino group at that position. The chitosan is obtained from natural sources of chitin, mostly from shells of crustaceans and squid pens from seafood processing plants.

It has been found that chitosan and its derivatives have antimicrobial activity against different groups of microorganisms such as bacteria and fungi. The bactericidal action of chitosan has been seen to be doue to destabilization of the cell membranes, causing loss of cellular content (14). There are many evidences of the effect of chitosan on plant pathogenic fungi. In particular, chitosan inhibits the germination of spores of phytopathogenic fungi (17), affects their growth (24, 16, 20, 17), inducing morphological and ultrastructural alterations in the hyphae (13) and causes reduced production of toxins (21).

### Description of Invention

The present invention allows to increasing sporulation of filamentous fungi using chitosan. These fungi may be used as biological control agents.

Spores are the main mechanism of inoculation of these fungi and therefore increasing the sporulation of fungal biological control agents allows an enhancement of the production of fungal inoculums for subsequent formulation and application.

Chitosan is a good inducer of sporulation of fungal biocontrol agents. Fungi growing in culture medium with chitosan show conidial production values more than 40 times higher than in the control without chitosan.

Chitosan used in these culture media has a number of advantages, which include but are not limited to the following:
- Does not affect the biological characteristics of conidia, it has been shown experimentally that the conidia of the entomopathogenic fungus *Beauveria bassiana* produced in medium with chitosan, show no difference in viability compared to control conidia.
- Does not affect the pathogenicity of the conidia, conidia derived from medium with chitosan did not show changes in their pathogenicity against insects when compared to control conidia.

Therefore, a first aspect of the invention relates to the use of chitosan to enhance the sporulation of entomopathogenic or nematophagous fungi where the nematophagous fungus is selected from the list that includes *Pochonia chlamydosporia, Paecilomyces lilacinus* or *Pochonia rubescens* and the entomopathogenic fungus is selected from the list that includes *Beauveria bassiana* or *Lecanicillium cf. psalliotae.*

Another aspect of the invention relates to a method for increasing the sporulation of entomopathogenic or nematophagous fungi that includes: the selection of the entomopathogenic or nematophagous fungus where the nematophagous fungus is selected from the list that includes *Pochonia chlamydosporia, Paecilomyces lilacinus* or *Pochonia rubescens* and the entomopathogenic fungus is selected from the list that includes *Beauveria bassiana* or *Lecanicillium cf. psalliotae,* its inoculation in a culture medium that includes at least chitosan, and the incubation of the fungus at proper conditions for fungal growth.

Another aspect of the invention relates a method to obtaining spores to be used as biological control agent which comprises: the selection of the entomopathogenic or nematophagous fungus, where the nematophagous fungus is selected from the list that includes *Pochonia chlamydosporia, Paecilomyces lilacinus* or *Pochonia rubescens* and the entomopathogenic fungus is selected from the list that includes *Beauveria bassiana* or *Lecanicillium cf. Psalliotae,* its inoculation in a culture medium that includes at least chitosan, incubation of the fungus at proper conditions for sporulation and isolation of the spores produced in the previous step.

In a preferred implementation the culture medium provides a chitosan concentration between 0.1 mg/ml and 2 mg/ml. In a more preferred implementation the culture medium provides a chitosan concentration between 0.5 mg/ml and 1.5 mg/ml. In a further preferred realization the culture medium provides a chitosan concentration of 1 mg/ml.

Throughout the specification and claims, the word "comprises" and its variants are not intended to exclude other technical features, additives, components or steps. For experts in the field, other objects, advantages and features of the invention will appear in part of the description and part of the practice of the invention. The following examples and figures illustrate the invention.

### Brief description of figures

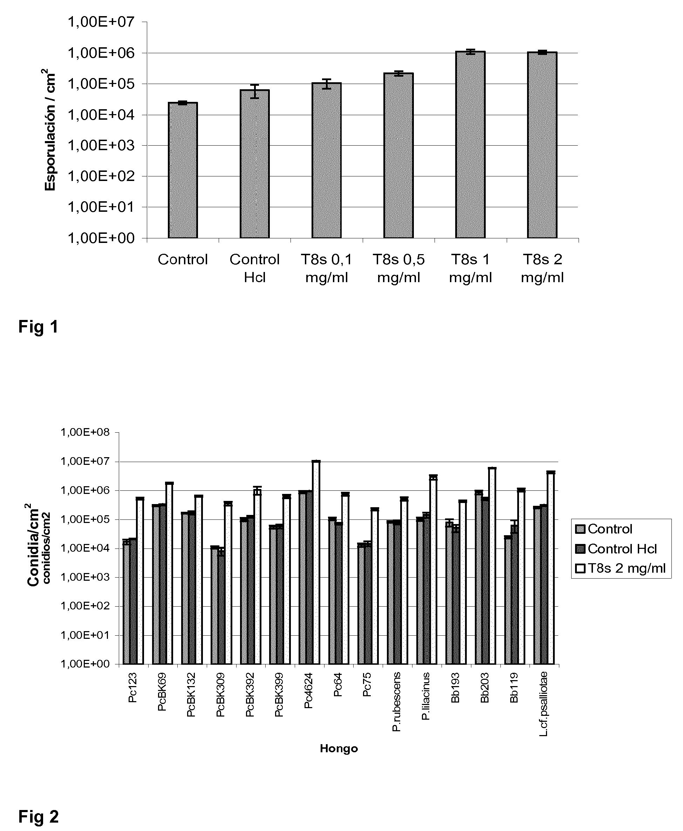

**Figure 1****:** Shows the result obtained in the sporulation of the fungus B. *bassiana* (isolate 119, colonies of 18 days) by adding increasing concentrations of chitosan to the culture medium (CMA, corn meal agar). The graph shows that increasing chitosan concentration is accompanied by an increase in sporulation, reaching a maximum value at 1 mg/ml of chitosan.
**Figure 2****:** Shows the effect of chitosan at 2mg/ml (in CMA) on sporulation of fungal biological control agents.

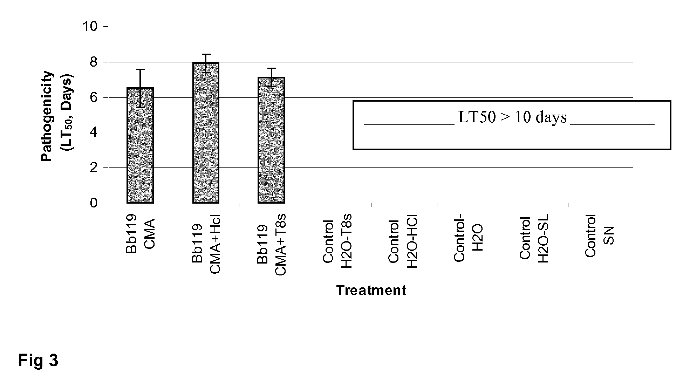

**Figure 3****:** LT50 value (median lethal time, time needed by an organism to kill the half of the population of the target organism) of the entomopathogenic fungus *Beauveria bassiana* (isolate 119) inoculated in *Galleria mellonella* at larval stages L3-L4. Treatments applied to *G*. *mellonella* larvae are:
   - *B. bassiana* 119: Bb119 CMA; Conidia from Bb119 grown in CMA culture media.
   - Bb119 CMA+HCl: Conidia from Bb119 grown in CMA with HCl.
   - Bb119 CMA+T8s: Conidia from Bb119 grown in CMA with 2 mg/ml chitosan.
   - Controls without *B. bassiana* 119: Control H₂O-T8s: consists in water from plates of CMA with chitosan.
   - H₂O-HCl Control: consists in water from plates of CMA with HCl.
   - H₂O Control: consists in water from CMA plates
   - H₂O-SL Control: water from CMA plates, without passing it trough glass wool.
   - SN control: Control without adding anything to the larvae.

### DETAILED STATEMENT OF PERFORMANCE OF MODES

### Example 1: Effect of chitosan in sporulation.

We studied the effect of chitosan on sporulation of nematophagous and entomopathogenic fungi. Fungi used were:
- *Pochonia chlamydosporia* (Pc), nematophagous fungus, 9 strains isolated from different geographical origin:
   P.c.123 (Seville, isolated from *Heterodea avenae*)
   P.c.BK69 (New Zealand)
   P.c.BK132 (Kenya)
   P.c.BK309 (Zimbabwe)
   P.c.BK392 (Cuba)
   P.c.BK 399 (China)
   P.C. 4624 (Perth, Australia)
   P.c.64 (Tarragona, isolated from *Meloidogyne* sp.)
   P.c75 (Valladolid, isolated from *Heterodea schachtii*)
- *Pochonia rubescens,* nematophagous fungus (Scotland, isolated from *Heterodea avenae*)*.*
- *Paecilomyces lilacinus,* nematophagous fungus
- *Beauveria bassiana,* entomopathogenic fungus, 3 strains isolated from different geographical origin:
   B.b119 (Orihuela, Alicante, isolated from Coleoptera)
   B.b193 (Valencia, isolated from *Rhynchophorus ferrugineus*),
   B.b203 (Elche, Alicante, isolated from *Rhynchophorus ferrugineus*).
- *Lecanicillium* cf. *psalliotae,* entomopathogenic fungus (Elche, Alicante, isolated from *Phoenicococcus marlatti*).

Chitosan used was T8s (Marine BioProducts GmbH), with a molecular weight of 70 KDa and a deacetylation degree of 79.6%.

Chitosan should be dissolved before its use. For this reason, the chitosan powder was dissolved in hydrochloric acid 0.25 M and adjusted to pH 5.6 with sodium hydroxide 1 M. The chitosan dissolution was dialysed in distilled water at 4 °C, with two changes per day over three days, to remove the salts present in chitosan or produced as a result of adjusting the pH of HCl to 5.6 with NaOH (2). After dialysis it was found that the pH of chitosan was stable at 5.6. Finally, the dissolved chitosan was sterilized by autoclave.
The culture medium supplemented with chitosan was prepared by using CMA culture medium (Corn Meal Agar, BBL). CMA was chosen to study the effect on sporulation because it is the culture medium in which the fungi studied showed the higher sporulation. Once sterilized by autoclave (20 minutes at 120 ° C) chitosan was added at the proper volume to obtain the final desired concentration of chitosan (13). Finally, culture medium was dispensed in sterile plastic Petri plates of 9 cm diameter. Plates with CMA without chitosan were used as control.

We performed a second control by adding 0.25 M hydrochloric acid, adjusted at pH 5.6 with sodium hydroxide and dialysed, to the culture medium. For this second control, it was used the same volume of HCl at pH 5.6 than the amount of chitosan used to prepare the highest concentration of chitosan (2 mg/ml). This second control was performed to rule out possible inhibitory effects due to sodium chloride produced by adjusting the pH of hydrochloric acid with sodium hydroxide.

The plates with the culture media described above were inoculated in the center with 5 mm plugs taken from the edge of colonies of different fungi growing actively, and incubated at 25 °C in darkness. There were three replicates per treatment.

When the colonies were between 18 and 21 days (close to reaching the edge of the plate) conidia were extracted and quantified.

To remove the conidia we worked in a laminar flow chamber, with material sterilized by autoclave, and the instrumental previously flamed. Sterile distilled water was added on the plate, conidia were collected with a micropipette and placed in sterile Eppendorf tubes of 1.5 ml. The concentration of conidia was estimated using a Neubauer chamber of 0,025 mm² area and 0.1 mm deep. In this way we calculated the number of conidia per cm².

In Figure 1 we can see that increasing chitosan concentration is accompanied by an increase in the sporulation of the fungus *B. bassiana* (isolate 119), reaching a maximum conidiation at 1 mg/ml chitosan.
The results of the effect of chitosan on fungal sporulation on fungal biological control agents are summarized in Table 1 and Figure 2. We can see that for both nematophagous and entomopathogenic fungi there is a clear increase in sporulation per cm² by chitosan. In some species or strains increased sporulation reaches more than 40 times compared to the control (*B. bassiana*, isolate 119).

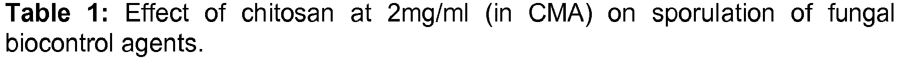

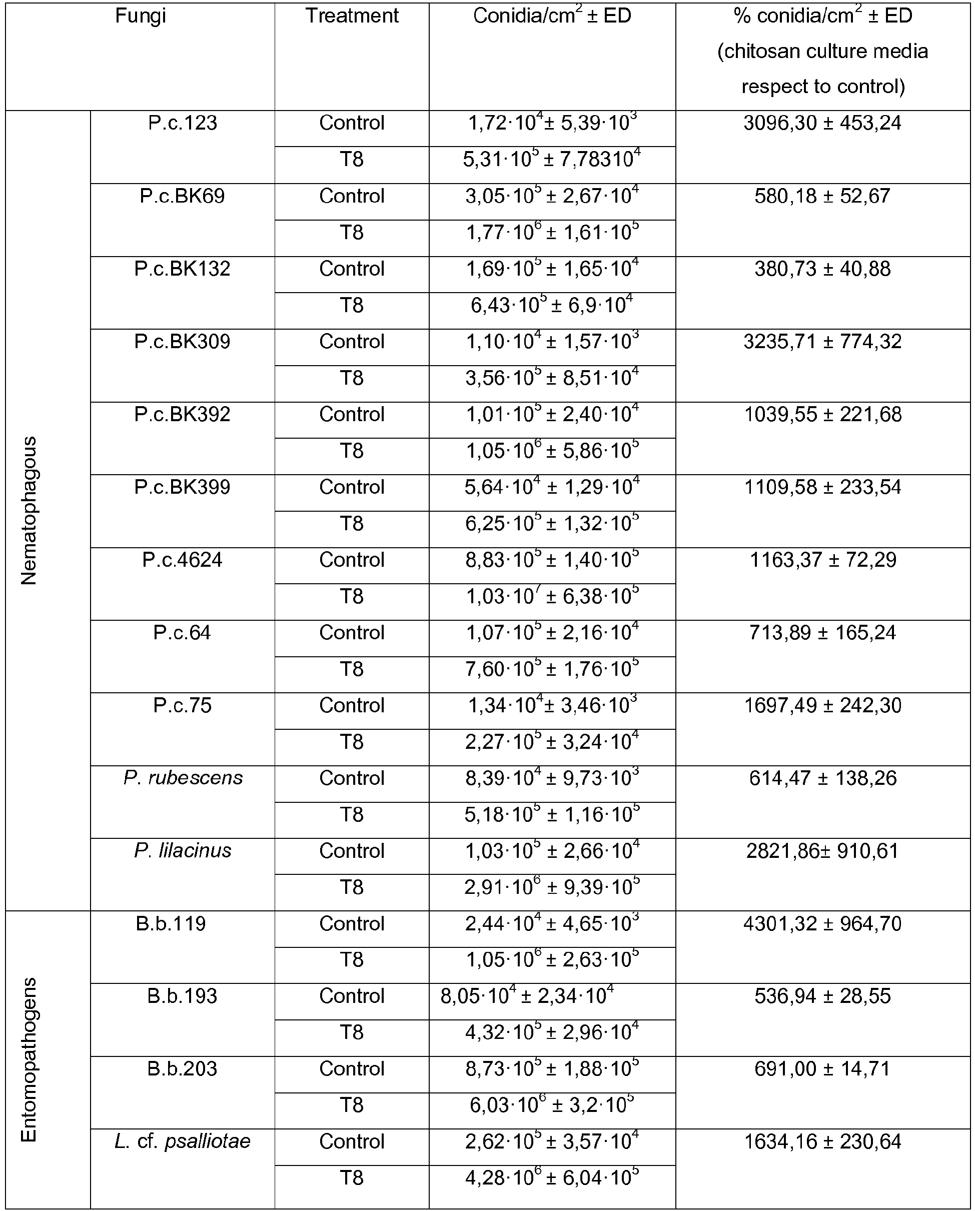

### Example 2: Viability of conidia

The viability of conidia from the fungi growing in medium with chitosan as described in Example 1 was checked comparing its germination percentages respect to the germination of conidia from the control plates (without chitosan).

For this purpose, the conidia were washed several times by centrifugation at 13,500 rpm for 5 minutes and resuspended in sterile distilled water. This will eliminate potential germination inhibitors produced by the conidia (22). Concentration was adjusted to 10⁶ conidia/ml using sterile distilled water. Conidia were placed on slides with ten wells of 6 mm in diameter (ICN Biomedicals) as follows: 25 µl were added to the suspension of conidia per well, with three replicates per treatment, and two wells per replicate (a total of six wells per treatment). The slides were placed inside a sterile petri plate on a sterilized wet piece of paper forming a wet chamber.

After 24 hours the number of germinated and non-germinated conidia were counted under a microscope, three areas at random from each well, and the percentage of germinated conidia was calculated. It was considered that the conidia had germinated when the germ tube length was longer than half the diameter of the conidia (17).

Finally, conidia were inoculated in Petri dishes with the rich in nutrients culture medium PDA (potato dextrose agar). The number of colonies was quantified throughout the following 72 hours.

No significant differences in the viability of conidia produced in chitosan-supplemented media with respect to the conidia produced in medium without chitosan were observed.

### Example 3: Effect of chitosan on the pathogenicity of the conidia.

To achieve the pathogenicity test, 15 larvae (L3-L4 stage), over 3 replicates, of the lepidopteran *Galleria mellonella* were used. Conidia of entomopathogenic fungus *Beauveria bassiana* (isolate 119) from plates without chitosan and from 2 mg/ml chitosan plates were used as inoculum. Conidia concentration was adjusted to 2·10⁶ conidia/ml with sterile distilled water.

5 larvae were placed in a petri dish. A drop (20 µl) of the conidial suspension at 2·10⁶ conidia/ml was added to each larvae, inoculating 4·10⁴ conidia per larva. The plates were closed to prevent escape of insects. Dead individuals of each of the treatments were scored every day for 10 days,.

Extracts without conidia from plates of CMA with chitosan, CMA with HCl and CMA were used as control. All these extracts were prepared passing the solution obtained from each plate through glass wool.

A fourth control was performed in which the solution obtained from CMA plates was not passed through glass wool.

Finally, a fifth control were nothing was added to CMA was performed (untreated insects).

With all the data obtained LT50 (median lethal time, time it takes a pathogen to kill half the population of the target organism) was calculated

The results of the pathogenicity tests are shown in Figure 3. No significant differences in the level of pathogenicity against *G*. *mellonela* were observed between conidia from the different treatments (fungus grown in plate CMA, CMA plate with chitosan or CMA plate with HCl), with LT50 values around the 6-7 days. The insect control treatments exceeded 10 days of survival, therefore it was not possible to estimate the LT50.

### Bibliographic references.

1. Asensio, L. (2004). Control biològic de plagues i malalties de palmeres per fongs entomopatògens. Tesis doctoral. Universidad de Alicante, 256pp.
2. Benhamou, N., Lafontaine, P.J., & Nicole, M. (1994). Seed treatment with chitosan induces systemic resistence to Fusarium crown and root rot in tomato plants. Phytopathology 84, 1432-1444.
3. Bordallo, J.J., Lopez-Llorca, L.V., Jansson, H. B., Salinas, J., Persmark, L. & Asensio, L. (2002). Colonization of plant roots by egg-parasitic and nematodo-trapping fungi. New Phytologist 154, 491-499.
4. Bourne, J. M., Kerry, B. R. & De Leij, F.A.A.M. (1996). The importance of the host plant on the interaction between root-knot nematodes (Meloidogyne spp.) and the nematophagous fungus Verticillium chlamydosporium Goddard. Biocontrol Science and Technology 6, 539-548.
5. De Leij, F. A. A. M., Kerry, B. R. & Dennehy, J. A. (1993). Verticillium chlamydosporium as a biocontrol agent for Meloidogyne incognita and M. hapla in pot and microplot tests, Nematologica 39, 115-126.
6. Ehteshamul, H. S., Zaki, M. J., Abid, M. & Ghaffar, A. (1994). Use of Verticillium chlamydosporium in the biological control of root-rot disease of chickpea. Pakistan Journal of Botany 26, 229-233.
7. Gomez-Vidal, S., Lopez-Llorca, L.V., Jansson, H.-B. & Salinas, J. (2006). Endophytic colonisation of date palm (Phoenix dactylifera L.) leaves by entomopathogenic fungi. Micron 37, 624-632.
8. Inglis, G.D., Goettel, M.S., Butt, T.M. & Strasser, H. (2001). Use of Hyphomycetous fungi for managing insect pests. En: Fungi as Biocontrol agents. CABI Publishing. p.p. 23-69.
9. Jacobs, H., Gray, S. N. & Crump, D. H. (2003). Interactions between nematophagous fungi and consequences for their potencial as biological agents for the control of potato cyst nematodes. Mycological Research 107, 47-56.
10. Jansson, H-B., & Lopez-Llorca, L.V. (2004). Control of nematode by fungi. Arora DK ed. Fungal biotechnology in agriculture, food, and environmental applications. Marcel Dekker, New York, p.p. 205-215.
11. Kerry, B. R. & Bourne, J.M. (1996). Importance of rhizosphere interactions in the biological control of plant-parasitic nematodes- a case of study using Verticillium chlamydosporium. Pesticide Science 47, 69-75.
12. Kendrick, B. (1985). The fifth kingdom. Micologue Publications. 363pp.
13. Laflamme, P., Benhamou, N., Bussieres, G., & Dessureault, M. (1999). Differential effect of chitosan on root rot fungal pathogens in forest nurseries. Canadian Journal of Botany 77, 1460-1468.
14. Liu, H., Du, Y., Wang, X. & Sun, L. (2004). Chitosan kills bacteria through cell membrane damage. International Journal of Food Microbiology 95, 145-155.
15. Lopez-Llorca, L.V., Olivares-Bernabeu, C.M., Salinas, J. & Jansson, H.B. (2002). Pre-penetration events in fungal parasites of nematode eggs. Mycological Research 106, 499-506.
16. Park, R.D., Jo, K.J., Jo,Y.Y., Jin,Y.L., Kim, K.Y., Shim, J.H., & Kim, Y.W. (2002). Variation of antifungal activities of chitosans on plant pathogens. Journal of Microbiology and Biotechnology 12, 84-88.
17. Plascencia-Jatomea, M., Viniegra, G., Olaya, R., Castillo-Ortega, M.M., & Shirai, K. (2003). Effect of chitosan and temperature on spore germination of Aspergillus niger. Macromolecular Bioscience 3, 582-586.
18. Poinar G.O. (1983). The natural history of nematodes. Prentice-Hall, Englewood Cliffs.
19. Quesada-Moraga, E., Landa, B.B., Muñoz-Ledesma, J., Jiménez-Diaz, R.M. & Santiago-Álvarez, C. (2006). Endophytic colonisation of Opium poppy, Papaver somniferum, by an entomopathogenic Beauveria bassiana strain. Mycopathologia 161, 323-329.
20. Rabea, E.I., Badawy, Mohamed E.T., Estevens, Christian V., Smagghe, G., & Steurbault, W. (2003). Review: Chitosan as antimicrobial agent: Applications and mode of action. BioMacromolecules 4, 1458-1465.
21. Reddy, M.V.B., Arul, J., Ait-Barka, E., Angers, P., Richard, C. & Castaigne, F. (1998). Effect of chitosan on growth and toxin production by Alternaria alternata f. sp. lycopersici. Biocontrol Science and Technology 8, 33-43.
22. Robinson, P.M. (1978). Practical fungal physiology. Wiley. 123pp.
23. Romero, D., Rivera, M. E., Cazorla, F. M., de Vicente, A. & Perez-Garcia, A. (2003). Effect of mycoparasitic fungi on the development of Sphaeroteca fusca in melon leaves. Mycological Research 107, 64-71.
24. Saniewska, A. (2001) . The effect of chitosan on limitation of growth and development of some pathogenic fungi for ornamental plants. Acta Agrobotanica 54 (1), 17-29.
25. Siddiqui, I. A. & Shaukat, S. S. (2003). Combination of Pseudomonas aeruginosa and Pochonia chlamydosporia for control of root-infecting fungi in tomato. Journal of Phytopathology 151 (4), 215-222.
26. Stirling. (1991). Biological control of plant parasitic nematodes. Progress, problems and prospects. CAB International, Wallingford.
27. Tikhonov, V.E., Stepnova, E.A., Babak, V.G., Yamskov, I.A., Palma-Guerrero,J., Jansson, H-B., Lopez-Llorca, L.V., Salinas, J., Gerasimenko, D.V., Avdienko, I.D. & Varlamov, V.P. (2006). Bactericidal and antifungal activities of a low molecular weight chitosan and its N-/2(3)-(dodec-2-enyl)succinoyl/- derivatives. Carbohydrate Polymers 64, 66-72.
28. Verdejo-Lucas, S., Sorbías, F.J., Ornat, C. & Galeano, M. (2003). Evaluating Pochonia chlamydosporia in a double-cropping system of lettuce and tomato in plastic houses infested with Meloidogyne javanica. Plant Pathology 52, 521-528.
29. Wagner, B.L. & Lewis, L.C. (2000). Colonization of Corn, Zea mays, by the Entomopathogenic Fungus Beauveria bassiana. Applied and Environmental Microbiology 66, 3468-3473.

## Claims

1. Use of chitosan to enhance sporulation of entomopathogenic or nematophagous fungi, where the nematophagous fungus is selected from the group consisting of: *Pochonia chlamydosporia, Paecilomyces lilacinus* or *Pochonia rubescens* and the entomopathogenic fungus is selected from the group consisting of: *Beauveria bassiana* or *Lecanicillium cf. psalliotae*.

2. Method to enhance sporulation of entomopathogenic or nematophagous fungi that includes:
A. Selection of the entomopathogenic or nematophagous fungus, where the nematophagous fungus is selected from the group consisting of: *Pochonia chlamydosporia, Paecilomyces lilacinus* or *Pochonia rubescens* and the entomopathogenic fungus is selected from the group consisting of: *Beauveria bassiana* or *Lecanicillium cf. psalliotae*
B. Inoculation of a culture medium that includes at least chitosan
C. Incubation at proper conditions for sporulation of fungi.

3. Method for obtaining spores for use as biological control agent that comprises:
A. Selection of the entomopathogenic or nematophagous fungus, where the nematophagous fungus is selected from the group consisting of: *Pochonia chlamydosporia, Paecilomyces lilacinus* or *Pochonia rubescens* and the entomopathogenic fungus is selected from the group consisting of: *Beauveria bassiana* or *Lecanicillium cf. psalliotae*
B. Inoculation in a culture medium that includes at least chitosan
C. Incubation at proper conditions for sporulation of fungi
D. Isolation of spores produced in the previous step.

4. Method according to any of claims 2 or 3, wherein the chitosan is at a concentration between 0.1 mg/ml and 2 mg/ml.

5. Method according to claim 4, wherein the chitosan is at a concentration between 0.5 mg/ml and 1.5 mg/ml.

6. Method according to claim 5, wherein the chitosan is at a concentration of 1 mg/ml.

## Patentansprüche

1. Verwendung von Chitosan zur Erhöhung der Sporulation von entomopathogenen oder nematophagen Pilzen, wobei der nematophage Pilz aus der Gruppe ausgewählt wird, welche aus Folgendem besteht: *Pochonia chlamydosporia, Paecilomyces lilacinus* oder *Pochonia rubescens,* und der entomopathogene Pilz aus der Gruppe ausgewählt wird, welche aus Folgendem besteht: *Beauveria bassiana* oder *Lecanicillium cf*. *psalliotae*.

2. Verfahren zur Erhöhung der Sporulation von entomopathogenen oder nematophagen Pilzen, einschließend:
A. die Auswahl vom entomopathogenen oder nematophagen Pilz, wobei der nematophage Pilz aus der Gruppe ausgewählt wird, welche aus Folgendem besteht: *Pochonia chlamydosporia*, *Paecilomyces lilacinus* oder *Pochonia rubescens,* und der entomopathogene Pilz aus der Gruppe ausgewählt wird, welche aus Folgendem besteht: *Beauveria bassiana* oder *Lecanicillium cf. psalliotae*
B. die Inokulation eines Kulturmediums, das mindestens Chitosan enthält,
C. die Inkubation unter geeigneten Bedingungen für die Sporulation von Pilzen.

3. Verfahren zur Erhaltung von Sporen für deren Verwendung als biologisches Bekämpfungsmittel, umfassend:
A. die Auswahl vom entomopathogenen oder nematophagen Pilz, wobei der nematophage Pilz aus der Gruppe ausgewählt wird, welche aus Folgendem besteht: *Pochonia chlamydosporia*, *Paecilomyces lilacinus* oder *Pochonia rubescens,* und der entomopathogene Pilz aus der Gruppe ausgewählt wird, welche aus Folgendem besteht: *Beauveria bassiana* oder *Lecanicillium cf. psalliotae*
B. die Inokulation in einem Kulturmedium, das mindestens Chitosan enthält,
C. die Inkubation unter geeigneten Bedingungen für die Sporulation von Pilzen
D. die Isolierung von Sporen, welche im vorhergehenden Schritt erzeugt wurden.

4. Verfahren nach einem der Ansprüche 2 oder 3, wobei das Chitosan bei einer Konzentration zwischen 0,1 mg/ml und 2 mg/ml vorliegt.

5. Verfahren nach Anspruch 4, wobei das Chitosan bei einer Konzentration zwischen 0,5 mg/ml und 1,5 mg/ml vorliegt.

6. Verfahren nach Anspruch 5, wobei das Chitosan bei einer Konzentration von 1 mg/ml vorliegt.

## Revendications

1. Utilisation du chitosane pour améliorer la sporulation de champignons entomopathogènes ou nématophages, où le champignon nématophage est choisi parmi le groupe constitué de : *Pochonia chlamydosporia*, *Paecilomyces lilacinus ou Pochonia rubescens* et le champignon entomopathogène est choisi parmi le groupe constitué de : *Beauveria bassiana* ou *Lecanicillium cf*. *psalliotae*.

2. Procédé d'amélioration de la sporulation de champignons entomopathogènes ou nématophages qui comporte :
A. la sélection du champignon entomopathogène ou nématophage, où le champignon nématophage est choisi parmi le groupe constitué de : *Pochonia chlamydosporia*, *Paecilomyces lilacinus* ou *Pochonia rubescens* et le champignon entomopathogène est choisi parmi le groupe constitué de : *Beauveria bassiana* ou *Lecanicillium cf*. *psalliotae*
B. l'inoculation d'un milieu de culture comportant au moins du chitosane
C. l'incubation dans des conditions appropriées pour la sporulation de champignons.

3. Procédé d'obtention de spores pour son utilisation comme agent de contrôle biologique comprenant :
A. la sélection du champignon entomopathogène ou nématophage, où le champignon nématophage est choisi parmi le groupe constitué de : *Pochonia chlamydosporia*, *Paecilomyces lilacinus* ou *Pochonia rubescens* et le champignon entomopathogène est choisi parmi le groupe constitué de : *Beauveria bassiana* ou *Lecanicillium cf*. *psalliotae*
B. l'inoculation dans un milieu de culture comportant au moins du chitosane
C. l'incubation dans des conditions appropriées pour la sporulation de champignons,
D. l'isolement des spores produites dans l'étape précédente.

4. Procédé selon l'une quelconque des revendications 2 ou 3, dans lequel le chitosane se trouve à une concentration entre 0,1 mg/ml et 2 mg/ml.

5. Procédé selon la revendication 4, dans lequel le chitosane se trouve à une concentration entre 0,5 mg/ml et 1,5 mg/ml.

6. Procédé selon la revendication 5, dans lequel le chitosane se trouve à une concentration de 1 mg/ml.
